# EUROPEAN PATENT SPECIFICATION

(11) **EP 4 066 738 B1**
(45) Date of publication and mention of the grant of the patent: **23.10.2024**
(21) Application number: 21165728.3
(22) Date of filing: 29.03.2021
(51) Int. Cl.: A61B 5/15, A61B 5/157, A61B 5/145, G01N 33/48

(54) **ELECTRONIC MODULE FOR A SAMPLE COLLECTION DEVICE AND SYSTEM COMPRISING THE SAME**
ELEKTRONISCHES MODUL FÜR EINE PROBENSAMMELVORRICHTUNG UND SYSTEM DAMIT
MODULE ÉLECTRONIQUE POUR UN DISPOSITIF DE COLLECTE D'ÉCHANTILLONS ET SYSTÈME LE COMPRENANT

(43) Date of publication of application: 05.10.2022
(73) Proprietor: Loop Medical SA, 1095 Lutry (CH)
(72) Inventor: QUEVAL, Arthur, 1095 Lutry (CH)
(74) Representative: DTS Patent- und Rechtsanwälte PartmbB

(56) References cited:
- WO-A1-2012/145322
- WO-A2-2009/107135
- US-A1- 2012 123 297
- US-A1- 2018 303 390
- US-A1- 2020 146 606

## Description

The present invention relates to an electronic module for a sample collection device according to the preamble of claim 1.

The present invention concerns also a system for extracting and collecting a sample of a fluid of a patient, e.g. blood, in particular capillary blood, comprising a sample collection device. Such an electronic module and such a system are known from WO 2009/107135 A2.

Venipuncture is a blood collection method where the vein is punctured by a hollow needle, and where blood is collected into a tube. This method allows collection of large and high quality blood samples into tubes. Several tubes can be filled during one blood sampling. Furthermore, these tubes are compatible with highly automated blood analyzers, which can analyze thousands of samples per day. These high throughputs capabilities answer the growing need to fast and clinical grade diagnostics at the lowest cost.

However, this method requires a healthcare professional (e.g. a nurse) with a specific qualification further than a dedicated infrastructure. Moreover, risks are associated with puncturing the vein: if the vein is fragile or if the gesture is not performed properly, it can result in a hematoma. There is also a risk of needle-stick injury, which may expose the healthcare professionals to blood-borne diseases.

On the other hand, the finger prick method consists in the incision of the skin at the fingertip using a lancet. A drop or a few drops of capillary blood can be collected into capillary tubes or into dedicated analytical devices (e.g. microfluidic devices, lab-on-chip, paper-based diagnostic tools, ...). While this technique does not require highly trained professional and can be performed by the patient himself, it is very difficult to collect blood above 100 µl and to perform many analyses per sample.

Moreover, the blood collected into glass capillaries or through other devices, cannot be analyzed by automated analyzers, used by central laboratories, which require to have a minimum dead volume of blood of 100 µl to 200 µl contained into a single tube.

In some instances, more blood, up to 0.5 ml, can be collected with the finger prick method. However, this requires to press and squeeze the finger in order to collect more blood. Squeezing too hard may result in hemolysis (damage of the red blood cells) and dilution of the blood sample by the interstitial fluid, contained in spaces between the tissue cells. For these reasons, and to keep a good blood quality, the use of finger prick is generally limited to the collection of small volumes of blood.

An improved system allowing simplifying collection of a fluid of a patient (e.g., blood) while keeping a high-quality standard for its analysis is known from WO2019220340, filed in the name of the same applicant. Said known extraction and collection system includes a first suction pack and a second suction pack, the first suction pack being arranged to be received by the second suction pack. Accordingly, a first chamber is defined between the first suction pack and the second suction pack, said chamber being placed under vacuum (e.g., in a manufacturing assembly line or in a healthcare facility). The second suction pack comprises a button, formed on one of its outer surfaces, and a piercing protrusion which is located below the button such that, once a user activates the button, the piercing protrusion pierces a membrane (e.g., an Aluminum membrane) provided in the first suction pack, thereby transferring the vacuum from the first vacuum chamber to a second chamber (i.e., a collection chamber).

However, in such a system, the user has to proactively stop collection of the fluid (e.g., blood) by detaching the system from a collection site on the skin of a patient.

In order to facilitate the user in monitoring blood collection progress, the second suction pack is formed of a transparent material and provided with filling lines.

Upon visually determining that a required amount of fluid for analysis has been collected, the user detaches the system from the collection site on the skin of the patient to stop fluid collection.

In case fluid collection is not stopped before the amount of collected fluid (e.g., blood) exceeds a certain amount, overflow and leakage of fluid may occur. This increases the risk of fluid spilling and dangerous cross-contamination.

On the other hand, in case fluid collection is stopped too soon, the amount of collected fluid can be insufficient for performing analysis.

An aim of the present invention is to propose an electronic element for a sample collection device allowing automatically and reliably detecting when a required amount of fluid for analysis is collected in a sample collection device.

According to the invention, these aims are achieved by means of an electronic module according to the attached independent claim 1.

In particular, an electronic module for a sample collection device is provided, the electronic module at least comprising:
- a measurement system for measuring a volume of collected fluid of a patient in real time and determining when a predefined amount of fluid has been collected, and
- means for providing a warning signal to a user when it is determined that the predefined amount of fluid has been collected.

The invention is based on the basic idea that, by measuring in real time the volume of collected fluid (e.g., blood) and providing a warning signal to the user when it is determined that a predefined amount of fluid has been collected, the user is enabled stopping fluid collection at an appropriate moment, thereby preventing occurrence of fluid overflow and leakage, at the same time ensuring that the collected amount of fluid is sufficient for performing analysis.

When properly used, the electronic module is not contaminated by blood. Thus, the electronic module can be reusable.

The electronic module is configured to be attached to, respectively detached from, the sample collection device.

The electronic module comprises a casing and a protruding body, formed on a surface of the casing.

The protruding body includes an activation button for operating a switch that activates the electronic module.

The protruding body includes a window for the arrangement of the measurement system.

The measurement system may comprise one or more sensors, preferably one or more optical sensors.

The casing and the protruding body can be formed in one piece through injection molding.

The protruding body may be eccentrically located on the surface on which it is formed. The electronic module may further comprise one or more of a battery, a microcontroller, an embedded firmware, and a communication unit for communicating with one or more external devices.

The communication unit is preferably a wireless communication unit.

The means for providing a warning signal may comprise an acoustic indicator and/or a visual indicator.

The visual indicator may comprise a light emitting unit, preferably a LED light emitting unit.

Accordingly, the user can be promptly warned when the required amount of fluid necessary for analysis has been collected, and fluid collection shall therefore be stopped.

The electronic module may further include a cover.

The visual indicator can be provided on the cover.

The cover may be circular in cross-section, and the visual indicator may be formed in the shape of a ring extending around the circumference of a main body portion of the cover.

The electronic module may further comprise means (e.g. sensors) for direct blood analysis.

The present invention further provides a system for extracting and collecting a sample of a fluid of a patient according to the attached independent claim 10. Specifically, the system at least comprises:
- a sample collection device, and
- the electronic module.

The sample collection device comprises an outer shell comprising a pocket configured for housing the protruding body of the casing of the electronic module. The protruding body and the pocket may be configured to have complementary asymmetrical shapes.

Accordingly, incorrect insertion of the protruding portion inside the pocket can be prevented.

The pocket may be configured to have rounded edges.

Furthermore, the pocket is equipped with an activation element, for example a pin. The activation element, e.g. the pin can be configured and arranged such that it activates the electronic module and especially the sensor, when the electronic module is inserted into the pocket.

The rounded edges provide guidance to the user and allow smooth insertion of the protruding body into the pocket.

Protruding portions can be formed on opposite sides of the pocket, and corresponding undercuts may be formed on a respective external surface of opposite sides of the protruding body, to allow snap fitting of the electronic module into the pocket.

The outer shell of the sample collection device can be made of a optically transparent material.

The invention will be better understood with the aid of the description of an embodiment given by way of example and illustrated by the figures. +

It is shown in
- **Fig. 1A**: a front view of a system for extracting and collecting a sample of a fluid of a patient equipped with an electronic module according to one embodiment of the invention;
- **Fig. 1B**: a side view of the system of Fig. 1A;
- **Fig. 1C**: a perspective view of the system of Fig. 1A;
- **Fig. 2**: a perspective view of an electronic module according to one embodiment of the invention, seen from a front side;
- **Fig. 3**: perspective view of the electronic module of Fig. 2, seen from a back side;
- **Fig. 4**: an exploded view of the electronic module of Fig. 2;
- **Fig. 5**: the electronic module and a detail of an outer shell of a sample collection device, provided with a pocket;
- **Fig. 6**: a detail of an undercut formed on an external surface of a side of a protruding body, formed on a casing of the electronic module;
- **Fig. 7**: a detail of the pocket formed on the outer shell of the sample collection device as a part of the snap-fit mechanism;
- **Fig. 8**: a horizontal cross section view of the protruding body coupled to the pocket through snap fitting;
- **Fig. 9**: a vertical cross section view of the protruding body coupled to the pocket through snap fitting;
- **Fig. 10**: a block diagram showing components of the electronic module;
- Fig. 11A: a front view of a PCB assembly of the electronic module, seen from a front side;
- Fig. 11B: a perspective view the PCB assembly of Fig. 11A, seen from a back side;
- Fig. 12: a flow diagram showing interaction between the components of the electronic device during a fluid collection process; and
- Fig. 13: a diagram showing optical properties of blood, here the absorption spectrum of human blood.

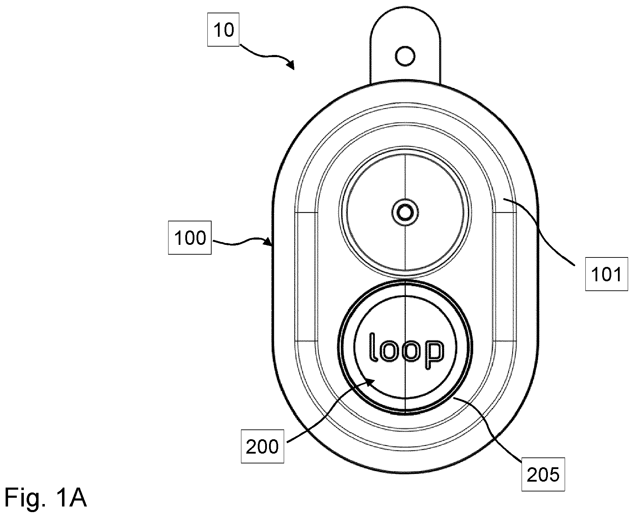

**Fig. 1A** shows a front view of a system 10 for extracting and collecting a sample of a fluid of a patient equipped with an electronic module 200 according to one embodiment of the invention.

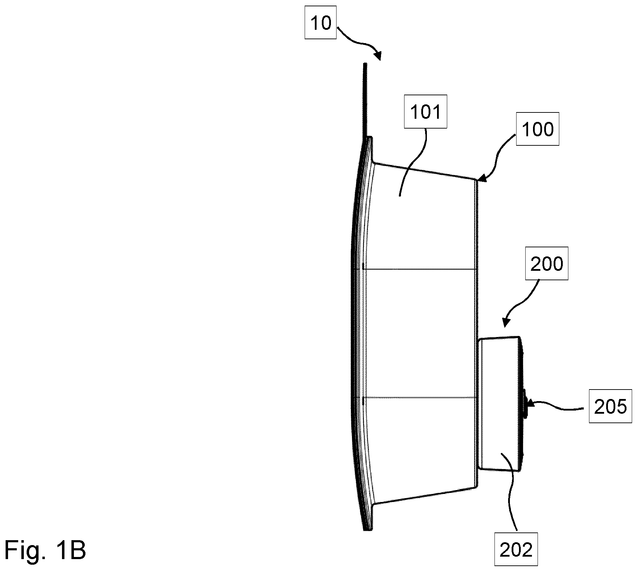

**Fig. 1B** shows a side view of the system of **Fig. 1A**.

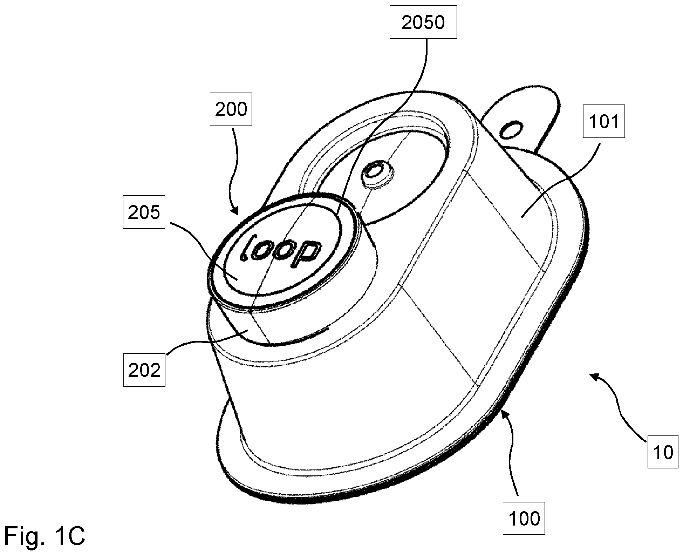

**Fig. 1C** shows a perspective view of the system of **Fig. 1A**.

**Figs. 1A to 1C** show different views of a system 10 for extracting and collecting a sample of a fluid of a patient according to an embodiment of the present invention.

The system 10 is equipped with an electronic module 200.

Further, the system 10 comprises a sample collection device 100.

The sample collection device 100 is provided for collection of a sample of a fluid, in particular a bodily fluid, e.g. blood, in particular capillary blood.

The electronic module 200 is detachable. It can be reused.

Further, it is possible that the electronic module can be mounted to the sample collection device 100 with a snap-fit mechanism as described below.

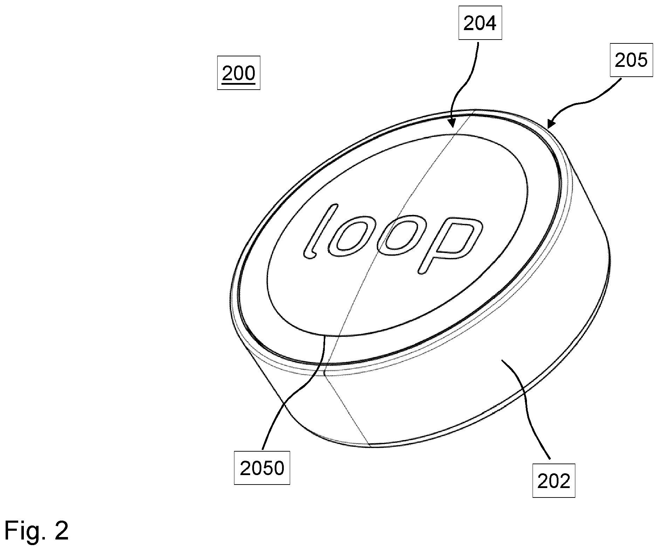

**Fig. 2** shows a perspective view of an electronic module 200 according to an embodiment of the invention, seen from a front side.

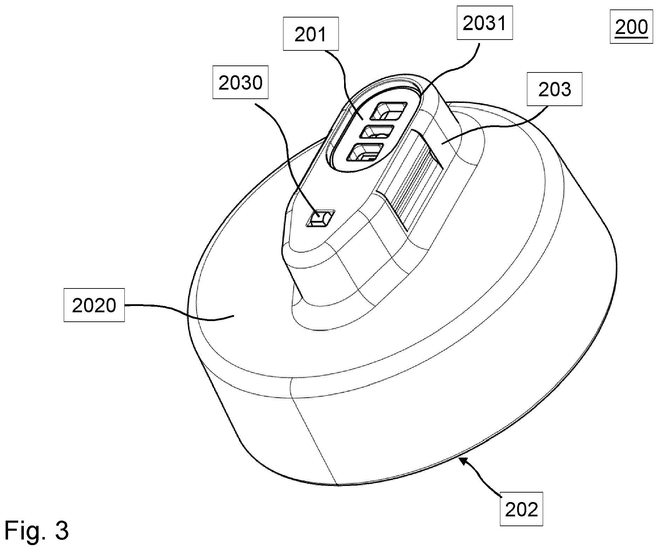

**Fig. 3** shows a perspective view of the electronic module 200, seen from a back side.

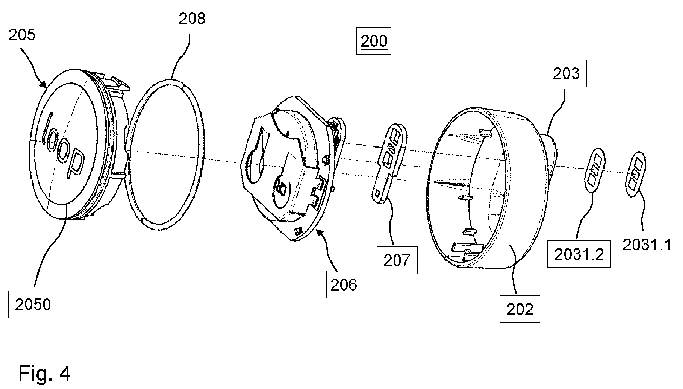

**Fig. 4** shows an exploded view of the electronic module 200.

As illustrated in **Fig. 3****,** the electronic module 200 comprises a measurement system 201 for measuring a volume of collected fluid (e.g., blood) of a patient in real time, and determining when a predefined amount of fluid for analysis has been collected.

Furthermore, the electronic module 200 includes means for providing a warning signal to a user when it is determined that the predefined amount of fluid has been collected.

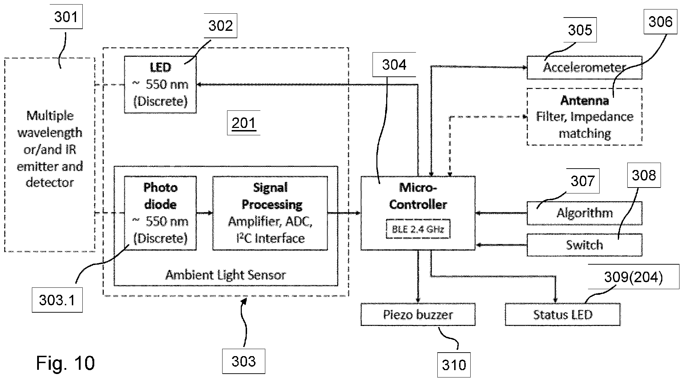

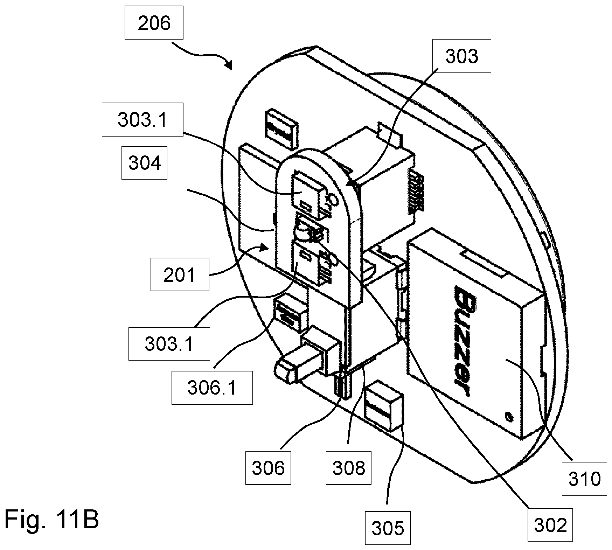

For example, the means for providing a warning signal may comprise an acoustic indicator 310 such as a buzzer **(****Fig. 10** **and** **Fig. 11B****).**

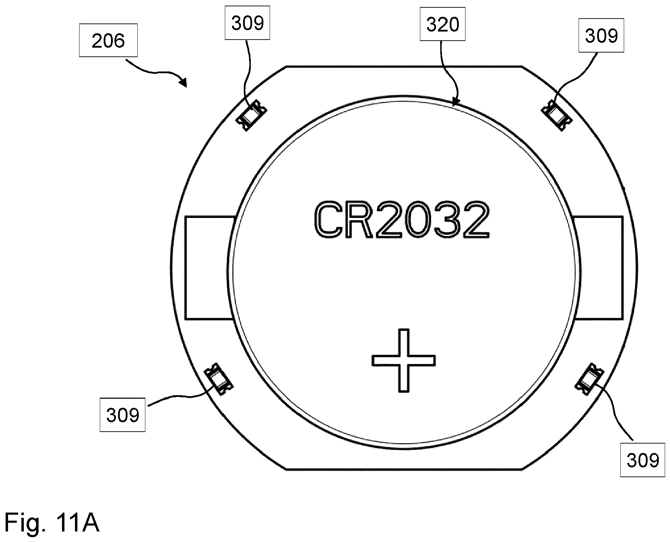

Additionally or alternatively, the means for providing a warning signal may comprise a visual indicator 204 (cf. **Fig. 2****)** provided with a light emitting unit, preferably a LED light emitting unit (status LED) 309 (see. **Fig. 10** and **Fig. 11A****).**

Conveniently, in the present embodiment, the electronic module 200 is reusable.

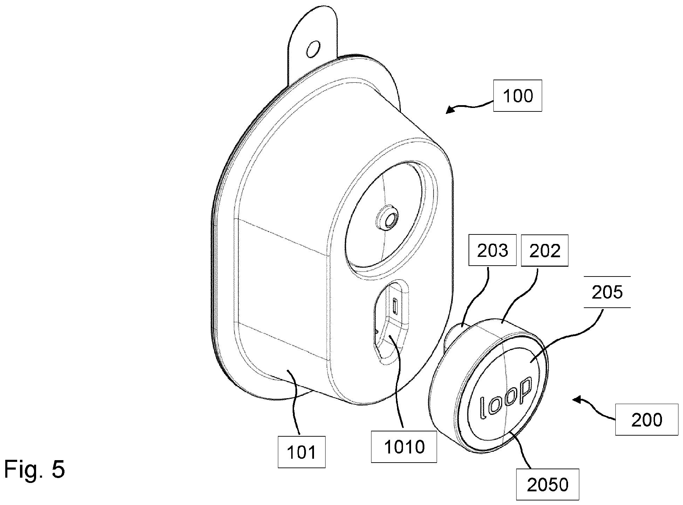

**Fig. 5** shows the electronic module 200 and a detail of an outer shell of a sample collection device 100, provided with a pocket.

Specifically, the electronic module 200 is configured to be attached to, respectively detached from, the sample collection device 100 (see **Fig. 5****).**

In the shown embodiment, the electronic module 200 further comprises a casing 202.

As shown in **Fig. 3****,** a protruding body 203 is formed on a surface 2020 of the casing 202.

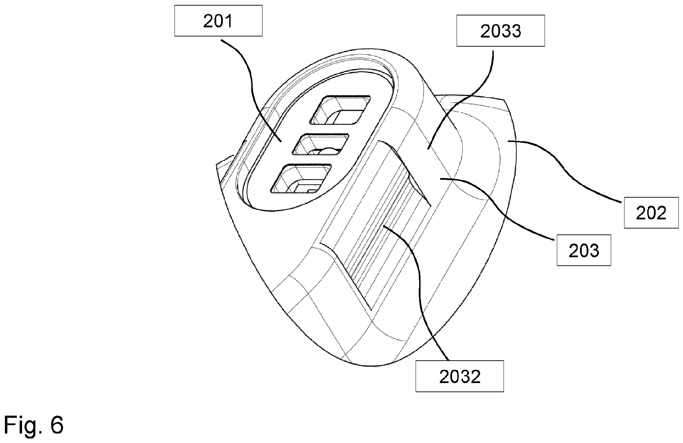

**Fig. 6** shows a detail of an undercut formed on an external surface of a side of the protruding body 203, formed on the casing 202 of the electronic module 200.

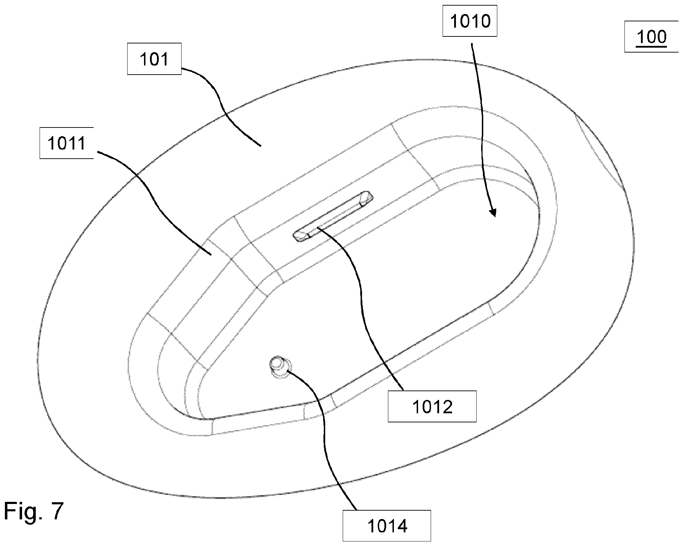

**Fig. 7** shows a detail of the pocket 1010 formed on the outer shell 101 of the sample collection device 100 as a part of the snap-fit mechanism.

In particular, in the pocket 1010 as part of the snap-fit mechanism protruding portions 1012 are formed on opposite sides of the pocket 1010.

The protruding portions 1012 can be provided by protruding ribs or any other suitable structure.

The pocket 1010 is equipped here with an activation element 1014, here in the form of a pin 1014. The pin 1014 is configured and arranged such that it activates the electronic module 200 and especially measuring system (e.g. here the sensor), when the electronic module 200 is inserted into the pocket.

The protruding body 203 comprises an activation button 2030, for operating a switch 308 (e.g., a mechanic switch) that activates the electronic module 200.

When the electronic module 200 is inserted into the pocket 1010, then the pin 1014 engages with the activation button 2030 and this way the electronic module is activated.

Additionally, the protruding body 203 comprises a window 2031 for the arrangement of the measurement system 201.

The window 2031 may be provided with a window overlay 2031.1 (see **Fig. 4****).**

The window overlay 2031.1 can be connected to the window 2031 through an adhesive layer (e.g., and adhesive film) 2031.2 (see also **Fig. 4****).**

The measurement system 201 includes one or more sensors, preferably one or more optical sensors.

The sensors can be advantageously arranged within support and protection means 207, for instance made of foam **(****Fig. 4****).**

**Fig. 10** shows a block diagram showing components of the electronic module 200 and how the components are related and connected to each other.

**Fig. 11A** shows a front view of a PCB assembly of the electronic module 200, seen from a front side.

**Fig. 11B** shows a perspective view the PCB assembly of **Fig. 11A****,** seen from a back side.

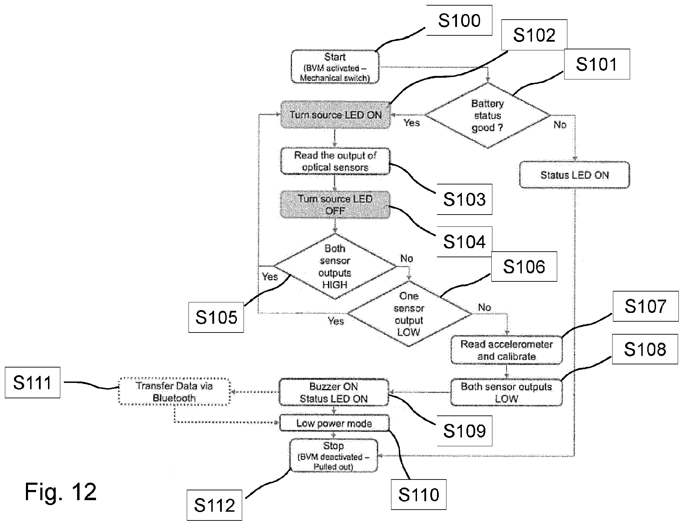

**Fig. 12** shows a flow diagram showing interaction between the components of the electronic device during a fluid collection process.

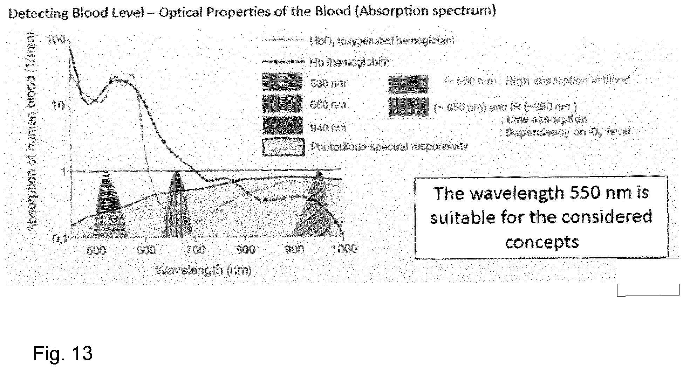

**Fig. 13** shows a diagram showing optical properties of blood, here the absorption spectrum of human blood.

The sensors (known in the state of the art), may be selected based on the optical properties of blood, shown in the diagram of **Fig. 13****.**

The sensors are configured to perform blood volume measurement.

The sensor on the PCB could be adapted to also detect the type of device (e.g. yellow cap, purple cap, ..., wherein the color indicated the type of measurement, which can be done with the electronic module) and adjust the collection parameters according to the type of the device.

The electronic module 200 can be further configured for direct blood analysis.

To this end, means for performing blood analysis such as direct blood analysis sensors can be provided in the electronic module 200.

In the shown embodiment, the electronic module 200 includes a battery 320.

The battery can be exchanged. It is possible that the user can exchange the battery. It is also possible that the housing of the electronic module is sealed, so that the exchange of the battery can be done by the manufacturer.

The battery 320 can be a coin cell, e.g. a CR2032 coin cell as in the shown embodiment. However, any other type of battery (either rechargeable or not rechargeable) may be used. Also, any other type of power source like a solar cell or the like is possible.

Moreover, the electronic module 200 includes a microcontroller 304.

The battery 320 and the microcontroller 304 are arranged on a PCB assembly 206 provided in the electronic module 200 **(****Fig. 4** and **Fig. 11A****-11B).**

The electronic module 200 of the present embodiment is further provided with an embedded firmware.

Still further, the electronic module 200 comprises a communication unit, preferably a wireless communication unit, more preferably a Bluetooth^{®} communication unit, for communicating with one or more external devices (not shown). Other communication possibilities and standards can also be used, such as WiFi, Zigbee and the like.

An exemplary architecture of the electronic module 200 is shown in **Fig. 10****.**

In the example of **Fig. 10****,** the electronic module 200 includes a sensor PCB 303.

The measurement system 201 is included in the sensor PCB 303.

In the present configuration, the measurement system 201 is provided with a pair of photodiodes 303.1.

Further, there is a source LED 302.

The source LED 302 is (operatively) connected to the microcontroller 304.

The sensor PCB 303 is (operatively) connected to the microcontroller 304.

The sensor PCB 303 may be further connected to a multiple wavelength and/or infrared emitter and detector unit 301.

The electronic module 200 further includes an accelerometer 305, operatively connected to the microcontroller 304.

The accelerometer 305 allows to identify the correct orientation of the device. In particular, which accelerometer 305 acceleration data can be retrieved, which allow to identify, whether the device is upside down or nor and to make a volume compensation, if the device is tilted. If the device is used upside down, the accelerometer detects it and may emit a sound and/or light signal to warn the user before activation of the device.

The electronic module 200 further includes the switch 308, operatively connected to the microcontroller 304.

The switch 308 is operated through the operating button 2030, so as to turn on/off the electronic module 200.

The microcontroller 304 controls operation of the LED light emitting unit 309 to emit a light signal to warn the user when it is determined that a predefined amount of fluid for analysis has been collected.

Similarly, the microcontroller 304 controls operation of the acoustic indicator (piezoelectric buzzer 310), to emit a warning signal to warn the user when it is determined that a predefined amount of fluid for analysis has been collected.

The electronic module 200 may further include an antenna 306.

Still further, the electronic module 200 may include an antenna filter 306.1 **(****Fig. 11B****).**

An algorithm 307 is implemented in the microcontroller 304 for controlling operation of the different components of the electronic module 200.

As shown in **Figs. 11A** **and** **11B****,** the sensor PCB 303, the source LED 302, the microcontroller 304, the accelerometer 305, the antenna 306, the antenna filter 306.1, the switch 308, the acoustic indicator (piezoelectric buzzer 310), and the LED light emitting unit 309, are arranged on the PCB assembly 206.

An example of the interactions between the different components of the electronic module 200 in a fluid collection process is shown in the flowchart of **Fig. 12****.**

The electronic module 200 is activated by operating the switch 308 via the operating button 2030 (S100).

Then, it is determined whether a battery is in a good status or not (S101).

If the battery is not in a good status, the process is ended (S112).

If the battery is in a good status, the source LED 302 is turned on (S102).

Then, an output of the pair of photodiodes (optical sensors) 303.1 is read by the microcontroller 304 (S103).

Then, the source LED 302 is turned off (S104).

After the source LED 302 is turned off, it is determined whether the output of both photodiodes 303.1 is high (S105).

If the output of both the photodiodes 303.1 is high, the process returns to step S102.

If the output of both photodiodes 303.1 is not high, it is determined whether the output of one of the photodiodes 303.1 is low (S106).

If the output of one of the photodiodes 303.1 is low (and the other sensor output is high), the process returns to step S102.

If the output of both of the photodiodes 303.1 is low, the microcontroller 304 processes data from the accelerometer 305, and performs calibration based on the processed data (S107, S108).

After performing calibration (S107) and determining that the output of both photodiodes 303.1 is low (S108), the piezoelectric buzzer 310 and the LED light emitting unit 309 are turned on (S109).

Then, the electronic module 200 is switched to a low power mode (S110).

Finally, the electronic module 200 is switched off and the process is ended (S112).

When the electronic module 200 is switched off and the process is ended (S112), the user can separate the electronic module 200 from the sample collection device 100 for further use.

Advantageously, data from the electronic module 200 can be transmitted to an external device (not shown) via the communication unit (preferably a wireless communication unit, more preferably a Bluetooth^{®} communication unit) (S111) before the electronic module 200 is turned off and the fluid collection process is ended (S112).

The electronic module 200 is capable of performing its functions with low energy consumption.

The electronic module 200 further includes a cover 205.

Specifically, in the shown embodiment, the cover 205 is circular in cross-section **(****Figs. 2** **and** **4****).**

In the shown embodiment, the visual indicator 204 is provided on the cover 205.

Specifically, as shown in **Fig. 2****,** the visual indicator 204 is formed in the shape of a ring extending around the circumference of a main body 2050 portion of the cover 205.

In the shown embodiment, the PCB assembly 206 is sandwiched between the casing 202 and the cover 205 **(****Fig. 4****).**

A plurality of LED light emitting units 309 are arranged on a front side of the PCB assembly 206 to illuminate the ring-shaped visual indicator 204 (**Fig. 11A**).

The ring-shaped visual indicator 204 is preferably made of a translucent material.

An O-ring 208 may be interposed between the cover 205 and the casing 202 (**Fig. 4**).

As an alternative, instead of providing the O-ring 208, an overmolding can be formed on the cover 205.

In the shown embodiment, the protruding body 203 is eccentrically located on the surface 2020 on which it is formed (**Fig. 3**).

Advantageously, the casing 202 and the protruding body 203 can be formed in one piece through injection moulding.

The sample collection device 100 comprises an outer shell 101 (as shown in **Figs. 1A to 1C****).**

In the shown embodiment, the outer shell 101 comprises a pocket 1010 (see **Fig. 5-7**), configured for housing the protruding body 203 of the casing 202 of the electronic module 200.

The protruding body 203 and the pocket 1010 are configured to have complementary asymmetrical shapes.

Accordingly, the protruding body 203 can only be inserted into the pocket 1010 in one direction.

Thus, incorrect insertion of the protruding body 203 into the pocket 1010 can be prevented.

In the shown embodiment, the pocket 1010 has rounded edges 1011 (see **Fig. 7**).

The rounded edges 1011 provide guidance to the user during insertion of the protruding body 203 into the pocket 1010.

Further, in the shown embodiment, protruding portions 1012 are formed on opposite sides of the pocket 1010 (see **Fig. 7**), and complementary undercuts 2032 are formed on a respective external surface 2033 of opposite sides of the protruding body 203 (see **Fig. 6**).

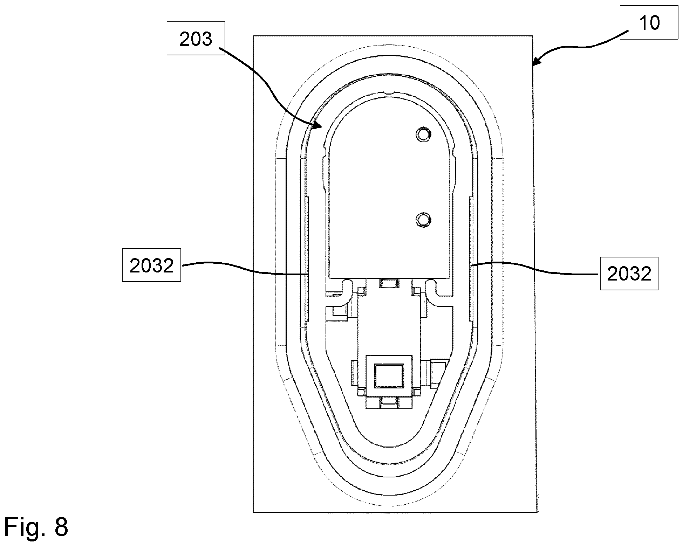

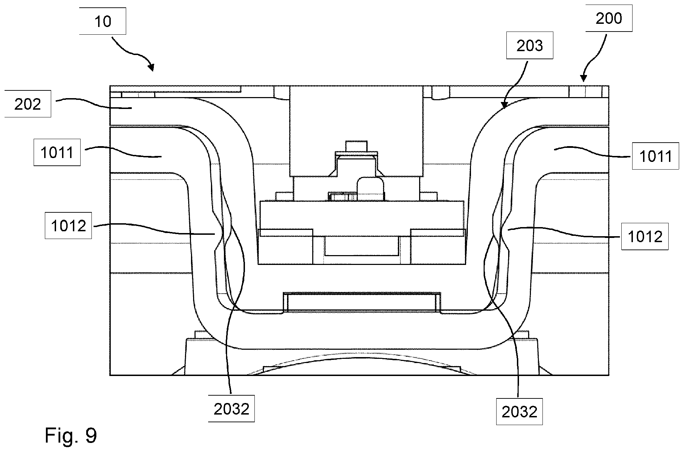

This allows easily and reliably coupling the protruding body 203 and the pocket 1010 through snap-fitting (see **Figs. 8-9**).

Advantageously, the outer shell 101 of the sample collection device 100 can be made of a transparent material to enable the user visually monitoring a blood collection progress.

Note that the example control and estimation routines included herein can be used with various sample collection device configurations and/or electronic module configurations. The control methods and routines disclosed herein may be stored as executable instructions in non-transitory memory and may be carried out by the control unit in combination with the various sensors, actuators, and other system hardware. The specific routines described herein may represent one or more of any number of processing strategies such as event-driven, interrupt-driven, multi-tasking, multi-threading, and the like. As such, various actions, operations, and/or functions illustrated may be performed in the sequence illustrated, in parallel, or in some cases omitted. Likewise, the order of processing is not necessarily required to achieve the features and advantages of the example embodiments described herein, but is provided for ease of illustration and description. One or more of the illustrated actions, operations and/or functions may be repeatedly performed depending on the particular strategy being used. Further, the described actions, operations and/or functions may graphically represent code to be programmed into non-transitory memory of the computer readable storage medium in the control unit/microcontroller, where the described actions are carried out by executing the instructions in a system including the various hardware components in combination with the electronic control unit (microcontroller).

### Reference signs used in the figures

- 10: System
- 100: Sample collection device
- 101: Outer shell
- 200: Electronic module
- 201: Measuring system
- 202: Casing
- 203: Protruding body
- 204: Visual indicator
- 205: Cover
- 206: PCB Assembly
- 207: Support and protection means
- 208: O-ring
- 301: Multiple wavelength and/or infrared emitter and detector
- 302: Source LED
- 303: Sensor PCB
- 303.1: Photodiode
- 304: Microcontroller
- 305: Accelerometer
- 306: Antenna
- 306.1: Antenna filter
- 307: Algorithm
- 308: Switch
- 309: LED light emitting unit (status LED)
- 310: Acoustic indicator (buzzer)
- 320: Battery
- 1010: Pocket
- 1011: Rounded edges of the pocket
- 1012: Protruding portion
- 1014: Activation element, Pin
- 2020: Surface of the casing on which the protruding body is formed
- 2030: Activation button
- 2031: Window
- 2031.1: Window overlay
- 2031.2: Adhesive layer
- 2032: Undercuts
- 2033: External surface
- 2050: Main body portion of the cover

## Claims

1. An electronic module (200) for a sample collection device (100), comprising:
a measurement system (201) for measuring a volume of collected fluid of a patient in real time and for determining when a predefined amount of fluid has been collected, and
means for providing a warning signal to a user when it is determined that the predefined amount of fluid has been collected,
wherein
the electronic module (200) further comprises a casing (202) and a protruding body (203) that is formed on a surface (2020) of the casing (202) for attaching to, respectively detaching from, the sample collection device (100), thereby providing for a reusable electronic module (200), **characterised in that**
the protruding body (203) comprises an activation button (2030) for operating a switch (308) that activates the electronic module (200), and a window (2031) for the arrangement of the measurement system (201).

2. The electronic module (200) according to claim 1,
**characterized in that**
the measurement system (201) comprises one or more sensors, preferably one or more optical sensors.

3. The electronic module (200) according to claim 1 or 2,
**characterized in that**
the protruding body (203) is eccentrically located on the surface (2020) on which it is formed.

4. The electronic module (200) according to claim 2 or 3,
**characterized in that**
the casing (202) and the protruding body (203) are formed in one piece through injection moulding.

5. The electronic module (200) according to one or more of the preceding claims, **characterized in that**
the electronic module (200) further comprises one or more of:
- a battery (320);
- a microcontroller (304);
- an embedded firmware, and
- a communication unit, preferably a wireless communication unit, for communicating with one or more external devices.

6. The electronic module (200) according to one or more of the preceding claims, **characterized in that**
the means for providing a warning signal comprises an acoustic indicator (310) and/or a visual indicator (204), the visual indicator (204) comprising a light emitting unit, preferably a LED light emitting unit.

7. The electronic module (200) according to claim 6,
**characterized in that**
the electronic module further comprises a cover (205), wherein the visual indicator (204) is provided on the cover (205).

8. The electronic module (200) according to claim 7,
**characterized in that**
the cover (205) is circular in cross-section, and the visual indicator (204) is formed in the shape of a ring extending around the circumference of a main body (2050) portion of the cover (205).

9. The electronic module (200) according to one or more of the preceding claims, **characterized in that**
the electronic module (200) further comprises means for direct blood analysis.

10. A system (10) for extracting and collecting a sample of a fluid of a patient, comprising a sample collection device (100),
and an electronic module (200) according to one or more of claims 1 to 9, wherein the sample collection device (100) is configured to be coupled to the electronic module (200), and
wherein
the sample collection device (100) comprises an outer shell (101) comprising a pocket (1010) configured for housing the protruding body (203) of the electronic module (200), wherein the pocket (1010) is equipped with an activation element (1014) configured and arranged for activating the electronic module (200), when the electronic module (200) is inserted therein.

11. The system (10) according to claim 10,
**characterized in that**
the protruding body (203) and the pocket (1010) are configured to have complementary asymmetrical shapes and/or the outer shell (101) of the sample collection device (100) is made of a transparent material.

12. The system (10) according to claim 10 or 11,
**characterized in that**
the pocket (1010) has rounded edges (1011) for providing guidance to the user during insertion of the protruding body (203) into the pocket (1010) and/or the pocket (1010) comprises an activation element (1014), for example a pin (1014).

13. The system (10) according to one or more of claims 10 to 12,
**characterized in that**
protruding portions (1012) are formed on opposite sides of the pocket (1010), and wherein corresponding undercuts (2032) are formed on a respective external surface (2033) of opposite sides of the protruding body (203), to allow snap fitting of the electronic module (200) into the pocket (1010).

## Patentansprüche

1. Elektronisches Modul (200) für eine Probensammelvorrichtung (100), aufweisend:
ein Messsystem (201) zum Messen eines Volumens eines gesammelten Fluids eines Patienten in Echtzeit und zum Feststellen, wann eine vordefinierte Menge an Fluid gesammelt worden ist, und
ein Mittel zum Bereitstellen eines Warnsignals an einen Benutzer, wenn festgestellt wird, dass die vordefinierte Menge an Fluid gesammelt worden ist,
wobei
das elektronische Modul (200) darüber hinaus ein Gehäuse (202) und einen vorstehenden Körper (203) aufweist, der an einer Oberfläche (2020) des Gehäuses (202) ausgebildet ist, um es an der Probensammelvorrichtung (100) anzubringen bzw. davon abzunehmen, wodurch ein wiederverwendbares elektronisches Modul (200) bereitgestellt wird, **dadurch gekennzeichnet, dass**
der vorstehende Körper (203) eine Aktivierungstaste (2030) zum Betätigen eines Schalters (308), der das elektronische Modul (200) aktiviert, und ein Fenster (2031) für die Anordnung des Messsystems (201) aufweist.

2. Elektronisches Modul (200) nach Anspruch 1,
**dadurch gekennzeichnet, dass**
das Messsystem (201) einen oder mehrere Sensoren aufweist, vorzugsweise einen oder mehrere optische Sensoren.

3. Elektronisches Modul (200) nach Anspruch 1 oder 2,
**dadurch gekennzeichnet, dass**
der vorstehende Körper (203) außermittig auf der Oberfläche (2020) sitzt, an der er ausgebildet ist.

4. Elektronisches Modul (200) nach Anspruch 2 oder 3,
**dadurch gekennzeichnet, dass**
das Gehäuse (202) und der vorstehende Körper (203) einstückig durch Spritzgießen ausgebildet sind.

5. Elektronisches Modul (200) nach einem oder mehreren der vorhergehenden Ansprüche,
**dadurch gekennzeichnet, dass**
das elektronische Modul (200) darüber hinaus ein oder mehrere der folgenden Elemente aufweist:
- eine Batterie (320);
- eine Mikrocontroller (304),
- eine eingebettete Software, und
- eine Kommunikationseinheit, vorzugsweise eine drahtlose Kommunikationseinheit, zum Datenaustausch mit einer oder mehreren externen Vorrichtungen.

6. Elektronisches Modul (200) nach einem oder mehreren der vorhergehenden Ansprüche,
**dadurch gekennzeichnet, dass**
das Mittel zum Bereitstellen eines Warnsignals eine akustische Anzeige (310) und/oder eine optische Anzeige (204) aufweist, wobei die optische Anzeige (204) eine Lichtemissionseinheit, vorzugsweise eine LED-Lichtemissionseinheit, aufweist.

7. Elektronisches Modul (200) nach Anspruch 6,
**dadurch gekennzeichnet, dass**
das elektronische Modul darüber hinaus eine Abdeckung (205) aufweist, wobei die optische Anzeige (204) an der Abdeckung (205) vorgesehen ist.

8. Elektronisches Modul (200) nach Anspruch 7,
**dadurch gekennzeichnet, dass**
die Abdeckung (205) im Querschnitt kreisförmig und die optische Anzeige (204) in Form eines Rings ausgebildet ist, der sich um den Umfang eines Hauptkörperabschnitts (2050) der Abdeckung (205) erstreckt.

9. Elektronisches Modul (200) nach einem oder mehreren der vorhergehenden Ansprüche,
**dadurch gekennzeichnet, dass**
das elektronische Modul (200) darüber hinaus ein Mittel zur direkten Blutanalyse aufweist.

10. System (10) zum Entnehmen und Sammeln einer Probe eines Fluids eines Patienten, das eine Probensammelvorrichtung (100) aufweist,
und ein elektronisches Modul (200) nach einem oder mehreren der Ansprüche 1 bis 9, wobei die Probensammelvorrichtung (100) dafür ausgelegt ist, mit dem elektronischen Modul (200) gekoppelt zu werden, wobei
die Probensammelvorrichtung (100) eine Außenhülle (101) aufweist, die ein Steckfach (1010) aufweist, das dafür ausgelegt ist, den vorstehenden Körper (203) des elektronischen Moduls (200) aufzunehmen, wobei das Steckfach (1010) mit einem Aktivierungselement (1014) ausgestattet ist, das dafür ausgelegt und eingerichtet ist, das elektronische Modul (200) zu aktivieren, wenn das elektronische Modul (200) darin eingesetzt ist.

11. System (10) nach Anspruch 10,
**dadurch gekennzeichnet, dass**
der vorstehende Körper (203) und das Steckfach (1010) so ausgelegt sind, dass sie komplementäre asymmetrische Formen haben, und/oder die Außenhülle (101) der Probensammelvorrichtung (100) aus einem transparenten Material besteht.

12. System (10) nach Anspruch 10 oder 11,
**dadurch gekennzeichnet, dass**
das Steckfach (1010) abgerundete Kanten (1011) zum Bereitstellen einer Führung für den Benutzer während des Einsetzens des vorstehenden Körpers (203) in das Steckfach (1010) hat und/oder das Steckfach (1010) ein Aktivierungselement (1014) wie zum Beispiel einen Stift (1014) aufweist.

13. System (10) nach einem oder mehreren der Ansprüche 10 bis 12,
**dadurch gekennzeichnet, dass**
vorstehende Abschnitte (1012) an gegenüberliegenden Seiten des Steckfachs (1010) ausgebildet sind, und wobei entsprechende Hinterschneidungen (2032) an einer jeweiligen Außenoberfläche (2033) von entgegengesetzten Seiten des vorstehenden Körpers (203) ausgebildet sind, um eine Schnappbefestigung des elektronischen Moduls (200) in dem Steckfach (1010) zu ermöglichen.

## Revendications

1. Module électronique (200) pour un dispositif de collecte d'échantillon (100), comprenant :
un système de mesure (201) prévu pour mesurer un volume de fluide collecté d'un patient en temps réel et pour déterminer lorsqu'une quantité prédéfinie de fluide a été collectée ; et
des moyens pour communiquer un signal d'avertissement à un utilisateur lorsqu'il est déterminé que la quantité prédéfinie de fluide a été collectée ;
dans lequel le module électronique (200) comprend en outre un carter (202) et un corps saillant (203) qui est formé sur une surface (2020) du carter (202) pour être fixé au dispositif de collecte d'échantillon (100) et respectivement en être détaché, fournissant ainsi un module électronique (200) réutilisable ;
**caractérisé en ce que** le corps saillant (203) comprend un bouton d'activation (2030) prévu pour actionner un commutateur (308) qui active le module électronique (200) ; et une fenêtre (2031) pour l'agencement du système de mesure (201).

2. Module électronique (200) selon la revendication 1, **caractérisé en ce que** le système de mesure (201) comprend un ou plusieurs capteurs, de préférence un ou plusieurs capteurs optiques.

3. Module électronique (200) selon la revendication 1 ou 2, **caractérisé en ce que** le corps saillant (203) est positionné de façon excentrée sur la surface (2020) sur laquelle it est formé.

4. Module électronique (200) selon la revendication 2 ou 3, **caractérisé en ce que** le carter (202) et le corps saillant (203) sont formés dans une pièce par moulage par injection.

5. Module électronique (200) selon l'une quelconque des revendications précédentes ou plusieurs d'entre elles, **caractérisé en ce que** le module électronique (200) comprend en outre un ou plusieurs éléments parmi :
- une batterie (320) :
- un microcontrôleur (304) :
- un microprogramme encastré ; et
- une unité de communication, de préférence une unité de communication sans fil, pour communiquer avec un ou plusieurs dispositifs externes.

6. Module électronique (200) selon l'une quelconque des revendications précédentes ou plusieurs d'entre elles, **caractérisé en ce que** les moyens prévus pour communiquer un signal d'avertissement comprennent un indicateur sonore (310) et/ou un visuel indicateur (204), l'indicateur visuel (204) comprenant une unité électroluminescente, de préférence une unité électroluminescente à DEL.

7. Module électronique (200) selon la revendication 6, **caractérisé en ce que** le module électronique comprend en outre un cache (205), dans lequel l'indicateur visuel (204) est prévu sur le cache (205).

8. Module électronique (200) selon la revendication 7, **caractérisé en ce que** le cache (205) est circulaire en section transversale et que l'indicateur visuel (204) prend la forme d'une bague s'étendant autour de la circonférence d'une partie de corps principal (2050) du cache (205).

9. Module électronique (200) selon l'une quelconque des revendications précédentes ou plusieurs d'entre elles, **caractérisé en ce que** le module électronique (200) comprend en outre des moyens pour réaliser l'analyse de sang.

10. Système (10) pour extraire et collecter un échantillon d'un fluide d'un patient, comprenant un dispositif de collecte d'échantillon (100) et un module électronique (200) selon l'une quelconque des revendications 1 à 9 ou plusieurs d'entre elles, dans lequel le dispositif de collecte d'échantillon (100) est configuré pour être couplé au module électronique (200) et dans lequel le dispositif de collecte d'échantillon (100) comprend une coque extérieure (101) comprenant une poche (1010) configurée pour loger le corps saillant (203) du module électronique (200), dans lequel la poche (1010) est équipée d'un élément d'activation (1014) configuré et agencé pour activer le module électronique (200) lorsque le module électronique (200) est inséré à l'intérieur.

11. Système (10) selon la revendication 10, **caractérisé en ce que** le corps saillant (203) et la poche (1010) sont configurés pour avoir des formes asymétriques complémentaires et/ou la coque extérieure (101) du dispositif de collecte d'échantillon (100) est fabriquée à partir d'un matériau transparent.

12. Système (10) selon la revendication 10 ou 11, **caractérisé en ce que** la poche (1010) a des bords arrondis (1011) pour guider l'utilisateur pendant l'insertion du corps saillant (203) dans la poche (1010) et/ou que la poche (1010) comprend un élément d'activation (1014), par exemple une broche (1014).

13. Système (10) selon l'une quelconque des revendications 10 à 12 ou plusieurs d'entre elles, **caractérisé en ce que** les parties saillantes (1012) sont formées sur les côtés opposés de la poche (1010) et dans lequel les contre-dépouilles correspondantes (2032) sont formées sur une surface externe respective (2033) des côtés opposés du corps saillant (203) pour permettre l'emboîtement par ajustement serré du module électronique (200) dans la poche (1010).
